# EUROPÄISCHE PATENTSCHRIFT

(11) **EP 1 004 290 B1**
(45) Veröffentlichungstag und Bekanntmachung des Hinweises auf die Patenterteilung: **02.02.2011**
(21) Anmeldenummer: 99121704.3
(22) Anmeldetag: 02.11.1999
(51) Int. Cl.: A61K 8/27, A61K 8/49, A61Q 17/04

(54) **Kosmetische oder pharmazeutische Zubereitungen enthaltend 1,3,5-Triazinderivate und Zinkoxid**
Cosmetic or pharmaceutical compositions containing 1,3,5-triazinderivatives and zinc oxide
Compositions cosmétiques ou pharmaceutiques contenant des dérivés de 1,3,5-triazine et d'oxide de zinc

(30) Priorität: 02.11.1998 DE 19850364; 27.08.1999 DE 19940889
(43) Veröffentlichungstag der Anmeldung: 31.05.2000
(73) Patentinhaber: BASF SE, 67056 Ludwigshafen (DE)
(72) Erfinder: Wünsch, Thomas, Dr., 67346 Speyer (DE); Westenfelder, Horst, 67435 Neustadt (DE)

(56) Entgegenhaltungen:
- EP-A- 0 685 224
- EP-A- 0 821 941
- EP-A- 0 893 119
- EP-A- 0 950 397
- TICHY S: "TRANSPARENT ZNO FOR SKIN- AND SUNPROTECTION" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 119, Nr. 8, 8. Juni 1993 (1993-06-08), Seiten 487-490, XP000370086 ISSN: 0942-7694

## Beschreibung

Die Erfindung betrifft kosmetische oder pharmazeutische Lichtschutzzubereitungen, enthaltend 1,3,5-Triazinderivate und Zinkoxid.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Ein vorteilhafter UV-B-Filter ist 2,4,6-Trianilino-p-(carbo-2'-ethyl-hexyl-1'-oxi)-1,3,5-triazin, der von der BASF Aktiengesellschaft unter der Warenbezeichnung Uvinul^{®} T150 vermarktet wird.

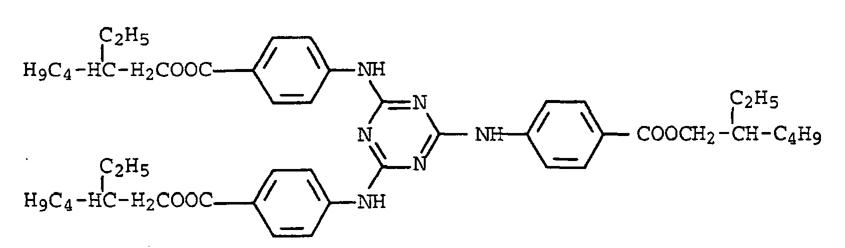

Uvinul^{®} T150 zeichnet sich durch gute UV-Absorptionseigenschaften mit einem außergewöhnlich hohen Extinktionskoeffizienten > 1500 bei 314 nm aus.

Ein Nachteil dieses UV-B-Filters ist jedoch die für viele kosmetische Anwendungen unzureichende Löslichkeit in kosmetischen Ölen.

Aufgrund der nur gering zu erzielenden Einsatzkonzentrationen an Uvinul^{®} T150 ist die Verwendung dieses UV-B-Filters für die Herstellung von kosmetischen oder pharmazeutischen Lichtschutzzubereitungen mit hohen Sonnenschutzfaktoren (SPF > 15) häufig eingeschränkt.

Zahlreiche Patentanmeldungen bzw. Patentschriften, u.a. US 5,489,431, DE-A-197 03 471, DE-A-196 32 913, DE-A-196 02 619, DE-A-196 35 057, DE-A-196 33 012, EP-A-0 685 223 beschreiben die Verwendung von Lösungsvermittlern, lipophilen Lösungsmitteln oder speziellen Kombinationen mit anderen UV-Absorbern zur Verbesserung der Löslichkeit von Uvinul^{®} T150.

In Dokument EP-A-0685224 werden Sonnenschutzmittel beschrieben, die das Triazin Uvinul T150, Benzol-1,4-di(3-methyliden-10-campher-sulfonsäure) und ein anorganisches Nanopigment auf Basis von Metalloxiden, insbesondere Titandioxid enthalten.

Dokument EP-A-0950397 offenbart in einem Beispiel eine Sonnenschutzformulierung, die 3% Octyltriazon (Uvinul T150) und 2% Zinkoxid enthält.

Dokument EP-A-0843995 offenbart die Verwendung von substituierten Benzazolen als UV-Absorber. In einem Beispiel wird eine Sonnenschutzlotion, die 1% Octyltriazon (Uvinul T150) und 7% Zinkoxid enthält, beschrieben.

Es bestand nun die Aufgabe, kosmetische oder pharmazeutische Lichtschutzzubereitungen bereitzustellen, die bei nur geringen Konzentrationen an UV-Absorbern, möglichst hohe Lichtschutzfaktoren aufweisen.

Diese Aufgabe wurde erfindungsgemäß gelöst durch kosmetische oder pharmazeutische Zubereitungen, enthaltend
a) 2,5 bis 5 Gew.-% 2,4,6-Trianilino-p-(carbo-2'-ethyl-hexyl-1'-oxi)-1,3,5-triazin der Formel Ia 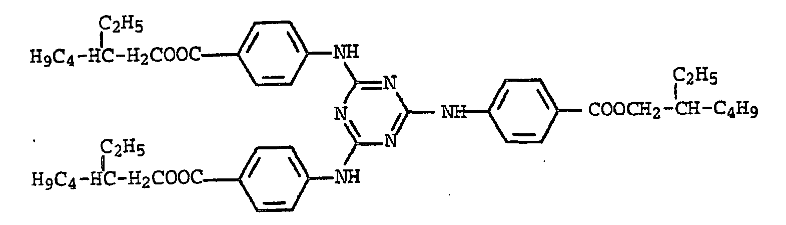 und
b) 3,5 bis 6 Gew.-% Zinkoxid, wobei das Zinkoxid ein Mikropigment mit einer Primärteilchengröße von 10 bis 60 nm ist.

Die Lichtschutzmittel enthaltenden kosmetischen und pharmazeutischen Zubereitungen sind in der Regel auf der Basis eines Trägers, der mindestens eine Ölphase enthält. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, Parabene wie Methylparaben, 1,2-Dibrom-2,4-dicyanobutan, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Als UV-Filtersubstanzen, die mit der erfindungsgemäßen Kombination aus 1,3,5-Triazinderivaten der Formel Ia und Zinkoxid angewandt werden können, kommen beliebige UV-A- und UV-B-Filtersubstanzen in Betracht. Beispielsweise sind zu nennen:

**Tabelle 1:**

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino]carbonyl]phenyl]amino]-1,3,5-triazin-2,4-diyl]diimino]bis-,bis(2-ethylhexyl)ester | 154702-15-5 |
| 30 | 2-(2H-Benzotriazol-2-yl)-4-methyl-6-[2-methyl-3-[1,3,3,3-tetramethyl-1-[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexyl)oxy] (Aniso Triazin) | 187393-00-6 |

Zum Schutz menschlicher Haare vor UV-Strahlen können die erfindungsgemäßen Lichtschutzzubereitungen als Shampoos, Lotionen, Gelen, Haarsprays, Aerosol-Schaumcremes oder Emulsionen u.a. zum Waschen, Färben sowie zum Frisieren der Haare verwendet werden.

Die UV-Filterwirkung der erfindungsgemäßen Zubereitungen kann auch zur Stabilisierung von Wirk- und Hilfsstoffen in kosmetischen und pharmazeutischen Formulierungen ausgenutzt werden.

Die erfindungsgemäßen Zubereitungen zeichnen sich durch ein besonders hohes Absorptionsvermögen im Bereich der UV-B-strahlung mit scharfer Bandenstruktur und hohen Lichtschutzfaktoren aus.

Insbesondere der hohe Lichtschutzfaktor der Zubereitungen, der bereits bei niedrigen Konzentrationen an UV-absorbierenden Wirkstoffen gemessen wurde, war überraschend.

So liegt der Lichtschutzfaktor der kosmetischen oder pharmazeutischen Formulierungen im Bereich größer 14, bevorzugt im Bereich größer 25. Entsprechende Werte lassen sich beispielsweise mittels einer Lichtschutzemulsion mit einem Gehalt an Uvinul^{®} T150 von 3 Gew.-% und einer Konzentration an Zinkoxid von 4 Gew.-% erzielen. Es ist aber auch möglich, die oben genannten Lichtschutzwerte durch Variation der Einsatzmengen an 1,3,5-Triazinderivaten der Formel Ia (im Bereich von 0,1 bis 10 Gew.-%) und Zinkoxid (im Bereich von 0,1 bis 10 Gew.-%) zu erzielen.

Wie aus Tabelle 2 zu sehen ist, zeigt die Kombination aus Uvinul^{®} T150 und Zinkoxid (Emulsion A) bezüglich des Lichtschutzfaktors synergistische Effekte, die deutlich über die additiven Effekte der jeweiligen Einzelkomponenten (Emulsion B und C) hinausgehen. Im Vergleich dazu ist der Lichtschutzfaktor einer vergleichbaren Uvinul^{®} T150/TiO₂-haltigen Emulsion (Emulsion D) kleiner als die Summe der Lichtschutzfaktoren der Einzelkomponenten (Emulsion C und E).

**Tabelle 2:**

| | | | | |
|---|---|---|---|---|
| | | | | |
| Emulsion¹⁾ | Uvinul^{®} T150 | TiO₂ | ZnO | Lichtschutzfaktor²⁾ |
| | | | | |
| A | 3 Gew.-% | - | 4 Gew.-% | 30 |
| B | - | - | 4 Gew.-% | 4 |
| C | 3 Gew.-% | - | - | 9 |
| D | 3 Gew.-% | 4 Gew.-% | - | 11 |
| E | - | 4 Gew.-% | - | 6 |
| | | | | |
| | | | | |

| | | | | |
|---|---|---|---|---|
| ¹⁾ Herstellung, siehe Beispiele; ²⁾ Bestimmt nach der Colipa Methode, beschrieben in Parfuem. Kosmet. (1994), 75(12), 856 | | | | |

Ein weiterer Vorteil der erfindungsgemäßen Zubereitungen liegt in der Tatsache, daß bereits mit sehr geringen Mengen an Triazinderivaten der Formel Ia (0,1 bis 1,5 Gew.-%) und Zinkoxid (ebenfalls 0,1 bis 1,5 Gew.-%) zufriedenstellende Lichtschutzwerte erhalten werden können.

Die folgenden Beispiele sollen die vorliegende Erfindung verdeutlichen, ohne sie einzuschränken.

Allgemeine Vorschrift zur Herstellung der erfindungsgemäßen Zubereitungen als Emulsionen

Die jeweiligen Phasen I und II wurden getrennt auf ca. 85°C erwärmt. Anschließend wurde Phase II unter Homogenisieren in Phase I eingerührt. Nach kurzem Nachhomogenisieren wurde die Emulsion unter Rühren auf Raumtemperatur abgekühlt und abgefüllt. Alle Mengenangaben sind auf das Gesamtgewicht der Zubereitungen bezogen.

### Beispiel 1

Emulsion A, enthaltend 3 Gew.-% Uvinul^{®} T150 und 4 Gew.-% Zinkoxid (Lichtschutzfaktor 30)

| | % | Rohstoff | INCI |
|---|---|---|---|
| | | | |
| I | 6,00 | Chremophor^{®} WO7 | PEG-7 Hydrogenated Castor Oil |
| | 0,30 | Chremophor^{®} RH 410 | PEG-40 Hydrogenated Castor Oil |
| | 2,00 | Elfacos^{®} ST9 | PEG-45/Dodecyl Glycol Copolymer |
| | 2,00 | Elfacos^{®} C26 | Hydroxyoctacosanyl Hydroxystearate |
| | 12,00 | Finsolv^{®} TN | Alkyl Benzoate |
| | 0,5 | Magnesiumstearat | Magnesium Stearate |
| | 0,5 | Aluminiumstearat | Aluminum Stearate |
| | 10,00 | Isopropylmyristat | Isopropyl Myristate |
| | 3,00 | Uvinul^{®} T150 | Octyl Triazone |
| | 0,5 | Konservierungsmittel | Preservative |
| | 5,0 | Witconol^{®} APM | PPG-3 Myristyl Ether |
| | 4,0 | Zinkoxid | Zinc Oxide |
| | | | |
| II | 0,3 | Konservierungsmittel | Preservative |
| | 5,0 | 1,2-Propylenglykol | Propylene Glycol |
| | 0,7 | Magnesiumsulfat | Magnesium Sulfate |
| | 48,20 | Wasser dem. | Aqua dem. |
| | | | |

### Vergleichsbeispiel 1

Emulsion B, enthaltend 4 Gew.-% Zinkoxid (Lichtschutzfaktor 4)

| | % | Rohstoff | INCI |
|---|---|---|---|
| | | | |
| I | 6,00 | Chremophor^{®} WO7 | PEG-7 Hydrogenated Castor Oil |
| | 0,30 | Chremophor^{®} RH 410 | PEG-40 Hydrogenated Castor Oil |
| | 2,00 | Elfacos^{®} ST9 | PEG-45/Dodecyl Glycol Copolymer |
| | 2,00 | Elfacos^{®} C26 | Hydroxyoctacosanyl Hydroxystearate |
| | 12,00 | Finsolv^{®} TN | Alkyl Benzoate |
| | 0,5 | Magnesiumstearat | Magnesium Stearate |
| | 0,5 | Aluminiumstearat | Aluminum Stearate |
| | 10,00 | Isopropylmyristat | Isopropyl Myristate |
| | 0,5 | Konservierungsmittel | Preservative |
| | 5,0 | Witconol^{®} APM | PPG-3 Myristyl Ether |
| | 4,0 | Zinkoxid | Zinc Oxide |
| | | | |
| II | 0,3 | Konservierungsmittel | Preservative |
| | 5,0 | 1,2-Propylenglykol | Propylene Glycol |
| | 0,7 | Magnesiumsulfat | Magnesium Sulfate |
| | 51,20 | Wasser dem. | Aqua dem. |

### Vergleichsbeispiel 2

Emulsion C, enthaltend 3 Gew.-% Uvinul^{®} T150 (Lichtschutzfaktor 9)

| | % | Rohstoff | INCI |
|---|---|---|---|
| | | | |
| I | 6,00 | Chremophor^{®} WO7 | PEG-7 Hydrogenated Castor Oil |
| | 0,30 | Chremophor^{®} RH 410 | PEG-40 Hydrogenated Castor Oil |
| | 2,00 | Elfacos^{®} ST9 | PEG-45/Dodecyl Glycol Copolymer |
| | 2,00 | Elfacos^{®} C26 | Hydroxyoctacosanyl Hydroxystearate |
| | 12,00 | Finsolv^{®} TN | Alkyl Benzoate |
| | 0,5 | Magnesiumstearat | Magnesium Stearate |
| | 0,5 | Aluminiumstearat | Aluminum Stearate |
| | 10,00 | Isopropylmyristat | Isopropyl Myristate |
| | 3,00 | Uvinul^{®} T150 | Octyl Triazone |
| | 0,5 | Konservierungsmittel | Preservative |
| | 5,0 | Witconol^{®} APM | PPG-3 Myristyl Ether |
| | | | |
| II | 0,3 | Konservierungsmittel | Preservative |
| | 5,0 | 1,2-Propylenglykol | Propylene Glycol |
| | 0,7 | Magnesiumsulfat | Magnesium Sulfate |
| | 52,20 | Wasser dem. | Aqua dem. |
| | | | |

### Vergleichsbeispiel 3

Emulsion D, enthaltend 3 Gew.-% Uvinul^{®} T150 und 4 Gew.-% Titandioxid (Lichtschutzfaktor 11)

| | % | Rohstoff | INCI |
|---|---|---|---|
| | | | |
| I | 6,00 | Chremophor^{®} WO7 | PEG-7 Hydrogenated Castor Oil |
| | 0,30 | Chremophor^{®} RH 410 | PEG-40 Hydrogenated Castor Oil |
| | 2,00 | Elfacos^{®} ST9 | PEG-45/Dodecyl Glycol Copolymer |
| | 2,00 | Elfacos^{®} C26 | Hydroxyoctacosanyl Hydroxystearate |
| | 12,00 | Finsolv^{®} TN | Alkyl Benzoate |
| | 0,5 | Magnesiumstearat | Magnesium Stearate |
| | 0,5 | Aluminiumstearat | Aluminum Stearate |
| | 10,00 | Isopropylmyristat | Isopropyl Myristate |
| | 3,00 | Uvinul^{®} T150 | Octyl Triazone |
| | 0,5 | Konservierungsmittel | Preservative |
| | 5,0 | Witconol^{®} APM | PPG-3 Myristyl Ether |
| | 4,0 | Tioveil^{®} MOTG | Titanium Dioxide |
| | | | |
| II | 0,3 | Konservierungsmittel | Preservative |
| | 5,0 | 1,2-Propylenglykol | Propylene Glycol |
| | 0,7 | Magnesiumsulfat | Magnesium Sulfate |
| | 48,20 | Wasser dem. | Aqua dem. |
| | | | |

### Vergleichsbeispiel 4

Emulsion E, enthaltend 4 Gew.-% Titandioxid (Lichtschutzfaktor 6)

| | % | Rohstoff | INCI |
|---|---|---|---|
| | | | |
| I | 6,00 | Chremophor^{®} WO7 | PEG-7 Hydrogenated Castor Oil |
| | 0,30 | Chremophor^{®} RH 410 | PEG-40 Hydrogenated Castor Oil |
| | 2,00 | Elfacos^{®} ST9 | PEG-45/Dodecyl Glycol Copolymer |
| | 2,00 | Elfacos^{®} C26 | Hydroxyoctacosanyl Hydroxystearate |
| | 12,00 | Finsolv^{®} TN | Alkyl Benzoate |
| | 0,5 | Magnesiumstearat | Magnesium Stearate |
| | 0,5 | Aluminiumstearat | Aluminum Stearate |
| | 10,00 | Isopropylmyristat | Isopropyl Myristate |
| | 0,5 | Konservierungsmittel | Preservative |
| | 5,0 | Witconol^{®} APM | PPG-3 Myristyl Ether |
| | 4,0 | Tioveil^{®} MOTG | Titanium Dioxide |
| | | | |
| II | 0,3 | Konservierungsmittel | Preservative |
| | 5,0 | 1,2-Propylenglykol | Propylene Glycol |
| | 0,7 | Magnesiumsulfat | Magnesium Sulfate |
| | 51,20 | Wasser dem. | Aqua dem. |
| | | | |

## Patentansprüche

1. Kosmetische oder pharmazeutische Zubereitungen, enthaltend
a) 2,5 bis 5 Gew.-% 2,4,6-Trianilino-p-(carbo-2'-ethyl-hexyl-1'-oxi)-1,3,5-triazin der Formel Ia 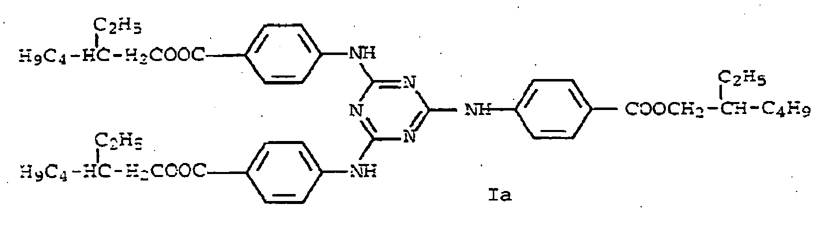 und
b) 3,5 bis 6 Gew.-% Zinkoxid, wobei das Zinkoxid ein Mikropigment mit einer Primärteilchengröße von 10 bis 60 nm ist.

2. Kosmetische oder pharmazeutische Zubereitungen nach Anspruch 1 mit einem Lichtschutzfaktor > 14.

3. Kosmetische oder pharmazeutische Zubereitungen nach Anspruch 1 oder 2 mit einem Lichtschutzfaktor > 25.

4. Kosmetische oder pharmazeutische Zubereitungen, definiert gemäß Anspruch 1, zur Anwendung zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

## Claims

1. A cosmetic or pharmaceutical preparation comprising
a) from 2.5 to 5% by weight of 2,4,6-trianilino-p-(carbo-2'-ethylhexyl-1'-oxy)-1,3,5-triazine of the formula Ia 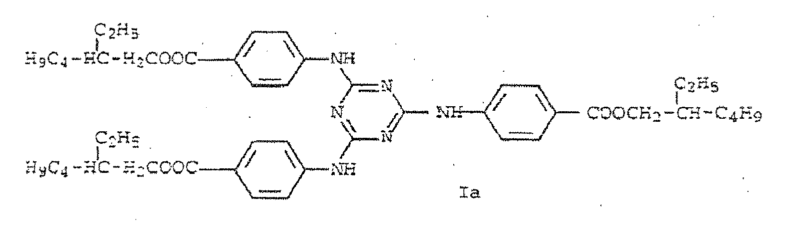 and
b) from 3.5 to 6% by weight of zinc oxide, where the zinc oxide is a micropigment having a primary particle size of from 10 to 60 nm.

2. The cosmetic or pharmaceutical preparation according to claim 1 having a sun protection factor of > 14.

3. The cosmetic or pharmaceutical preparation according to claim 1 or 2 having a sun protection factor of > 25.

4. The cosmetic or pharmaceutical preparation, defined as in claim 1, for use for protecting the human skin or human hair from solar rays, alone or together with compounds which absorb in the UV region and are known per se for cosmetic and pharmaceutical preparations.

## Revendications

1. Préparations cosmétiques ou pharmaceutiques, contenant
a) 2,5 à 5 % en poids de 2,4,6-trianilino-p-(carbo-2'-éthyl-hexyl-1'-oxy)-1,3,5-triazine de formule Ia 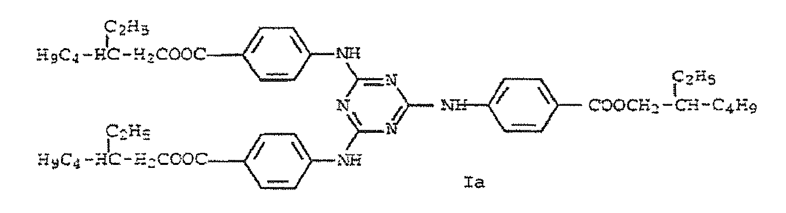 et
b) 3,5 à 6 % en poids d'oxyde de zinc, l'oxyde de zinc étant un micropigment ayant une taille de particule primaire de 10 à 60 nm.

2. Préparations cosmétiques ou pharmaceutiques selon la revendication 1, ayant un facteur de photoprotection > 14.

3. Préparations cosmétiques ou pharmaceutiques selon la revendication 1 ou 2, ayant un facteur de photoprotection > 25.

4. Préparations cosmétiques ou pharmaceutiques définies selon la revendication 1, pour l'utilisation dans la protection de la peau humaine ou des cheveux humains contre les rayons solaires, seules ou conjointement avec des composés absorbant dans la région UV, connus en soi pour des préparations cosmétiques et pharmaceutiques.
